# EUROPÄISCHE PATENTSCHRIFT

(11) **EP 2 926 897 B1**
(45) Veröffentlichungstag und Bekanntmachung des Hinweises auf die Patenterteilung: **07.08.2019**
(21) Anmeldenummer: 15162339.4
(22) Anmeldetag: 02.04.2015
(51) Int. Cl.: B01J 20/34

(54) **VORRICHTUNG ZUR RÜCKGEWINNUNG VON HALOGENIERTEN KOHLENWASSERSTOFFEN**
DEVICE FOR RECOVERY OF HALOGENATED HYDROCARBONS
DISPOSITIF DE RÉCUPÉRATION D'HYDROCARBURES HALOGÉNÉS

(30) Priorität: 03.04.2014 DE 202014101587 U
(43) Veröffentlichungstag der Anmeldung: 07.10.2015
(73) Patentinhaber: ZeoSys GmbH, 13088 Berlin (DE)
(72) Erfinder: Welke, Hartmut, 16356 Ahrensfelde (DE)
(74) Vertreter: Schulz Junghans Patentanwälte PartGmbB

(56) Entgegenhaltungen:
- EP-A1- 2 644 572
- WO-A1-2007/093640
- WO-A1-2013/106657
- DE-A1- 10 118 768

## Beschreibung

Die Erfindung betrifft eine Vorrichtung zur Rückgewinnung von halogenierten Kohlenwasserstoffen.

### Hintergrund

Aus dem Stand der Technik sind verschiedene Technologien zur Rückgewinnung von Inhalationsanästhetika bekannt.

Das Dokument WO 99/22845 A2 betrifft ein Verfahren und eine Vorrichtung zur Rückgewinnung von Gasen, insbesondere Narkosegasen, aus Zeolithen. Ein Absorber wird mit Zeolith gefüllt. An den Absorber ist eine Chemievakuumpumpe angeschlossen, die einen Unterdruck erzeugt. Anschließend wird der Absorber mittels eines Heizelements erhitzt und so das Narkosegas aus dem Zeolith ausgetrieben

Das Dokument WO 2007/093640 A1 offenbart eine Filterpatrone zur Rückgewinnung von Kohlenwasserstoffen und ein entsprechendes Verfahren. Die Filterpatrone enthält Zeolithe, welche Narkosegase an sich binden. Zur Rückgewinnung des Narkosegases wird Wasserdampf durch die Filterpatrone geleitet.

Ein ähnliches Verfahren zum Wiedergewinnen von Narkosegasen aus einer Filterpatrone ist im Dokument DE 101 18 768 A1 beschrieben.

Das Dokument WO 2013/106657 A1 offenbart eine Wasserfiltriereinrichtung zum Herstellen von medizinischem Wasser zur Injektion.

### Zusammenfassung

Aufgabe ist es, verbesserte Technologien zur Rückgewinnung von halogenierten Kohlenwasserstoffen anzugeben.

Die Aufgabe wird durch die Vorrichtung nach Anspruch 1 gelöst. Weitere Ausführungsformen sind Gegenstand von abhängigen Ansprüchen.

Nach einem Aspekt ist eine Vorrichtung zur Rückgewinnung von halogenierten Kohlenwasserstoffen gebildet, mit einem Desorptionsbehälter, der ein Sorbat aufnimmt, welches die halogenierten Kohlenwasserstoffen umfasst, einem Dampferzeuger, welcher konfiguriert ist, aus dem Dampferzeuger zugeführtem Wasser Wasserdampf zu erzeugen und den erzeugten Wasserdampf in den Desorptionsbehälter einzuleiten, derart, dass die halogenierten Kohlenwasserstoffe von dem Sorbat desorbieren und von dem Wasserdampf aufgenommen werden, einer Kühleinrichtung, die konfiguriert ist, den mit halogenierten Kohlenwasserstoffen versetzten Wasserdampf zu kühlen, derart, dass ein Kondensat gebildet wird, und einem Auffangbehälter, welcher das Kondensat aufnimmt.

Es ist eine Vorrichtung geschaffen, die eine effiziente Rückgewinnung der halogenierten Kohlenwasserstoffe ermöglicht.

Die halogenierten Kohlenwasserstoffe können beispielsweise Inhalationsanästhetika sein, insbesondere Narkosegase wie Sevofluran, Enfluran, Isofluran, Haloethan und Desfluran.

Das Sorbat weist ein Sorptionsmittel auf, an welchem die halogenierten Kohlenwasserstoffe adsorbiert sind. Das Sorptionsmittel kann mikroporös sein. Das Sorptionsmittel kann ein Material aus der Klasse der Zeolithe sein. Zeolithe sind kristalline Alumosilikate, die in zahlreichen Modifikationen in der Natur vorkommen, aber auch synthetisch hergestellt werden können. Beispielsweise können Si-reiche Zeolithe als Sorptionsmittel verwendet werden, bevorzugt mit einem Si:Al Verhältnis von größer als 180:1 (entspricht einem SiO₂:Al₂O₃ Verhältnis von 360:1). Alternativ kann Aktivkohle als Sorptionsmittel eingesetzt werden.

Es kann vorgesehen sein, dass der Desorptionsbehälter einen oder mehrere Adsorbentienbehälter aufnimmt, beispielsweise zwei. Das Sorbat wird dann in den einen oder mehrere Adsorbentienbehälter gefüllt. Der oder die Absorbentienbehälter können am Boden und / oder an einem Deckel mit einem Filtergewebe gebildet sein. Das Filtergewebe kann eine Porengröße von 40 µm aufweisen.

Der Dampferzeuger kann mehrere Heizelemente aufweisen, welche abhängig vom Leistungsbedarf einzeln zuschaltbar sein können, um den Dampfdruck zu regeln. Der Dampferzeuger kann mit einer Füllstandskontrolle gebildet sein. Der Dampferzeuger wird beispielsweise abgeschaltet, wenn ein oberer oder ein unterer Schaltpunkt der Füllstandskontrolle erreicht wird.

Die Kühleinrichtung kann zwei Komponenten aufweisen, einen Vorkühler und einen Zwischenkühler. Der Vorkühler und der Zwischenkühler können hintereinander angeordnet sein. Mit dem Vorkühler wird neu eingespeistes Wasser für den Dampferzeuger vorgewärmt. Der Zwischenkühler dient zum Abkühlen des mit Kohlenwasserstoff versetzten Wasserdampfes auf eine gewählte Temperatur, sodass sich ein Kondensat bildet. Des Weiteren kann ein Nachkühler vorgesehen sein. Zwischen dem Zwischenkühler und dem Nachkühler kann ein Filter angeordnet sein. Der Filter kann eine Porengröße von 5 µm haben. Eine Filterkerze des Filters kann aus Kunststoff bestehen, beispielsweise aus Polypropylen. Ein Gehäuse des Filters kann aus Edelstahl bestehen, beispielsweise aus Edelstahl mit der Werkstoffnummer 1.4404. Der Nachkühler kann mit Wasser aus einer öffentlichen Wasserversorgung betrieben werden.

In dem Auffangbehälter sinken die desorbierten Kohlenwasserstoffe (Desorbat) nach unten, da sie schwerer als Wasser sind. Der Auffangbehälter kann mit einer Füllstandskontrolle gebildet sein. Es kann ein weiterer Auffangbehälter vorgesehen sein, der ebenfalls eine Füllstandskontrolle aufweisen kann. Der weitere Auffangbehälter kann mit dem Auffangbehälter verbunden sein. Es kann eine Verbindung der beiden Auffangbehälter in einem oberen Bereich gebildet sein, welche das Abtrennen des Wassers ermöglicht. Der Auffangbehälter und / oder der weitere Auffangbehälter können mit einem Ventil zur Entnahme des Desorbats gebildet sein.

Die Vorrichtung weist des Weiteren eine Enthärtungseinrichtung auf, welche dem Dampferzeuger vorgelagert ist und welche konfiguriert ist, den Härtegrad des zugeführten Wasser zu verringern. Der Härtegrad des Wassers bezeichnet die Äquivalentkonzentration von im Wasser gelösten Ionen, insbesondere Ionen der Erdalkalimetalle. Unter Umständen werden hierfür auch deren anionischer Partner berücksichtigt. Die Härte des Wassers erhöhen beispielsweise Calcium- und Magnesiumionen sowie Strontium- und Bariumionen ("Härtebildner"). Die gelösten Härtebildner können unlösliche Verbindungen bilden, vor allem Kalk und Kalkseifen. Die Vorrichtung kann beispielsweise an eine öffentliche Wasserversorgung angeschlossen sein. Je nach Region ist das von der Wasserversorgung bereitgestellte Wasser unterschiedlich hart. Zur Verringerung des Härtegrads des Wassers können beispielsweise die an sich bekannten Verfahren der Entcarbonisierung und / oder der Enthärtung durch Ionenaustausch eingesetzt werden.

Der Enthärtungseinrichtung kann ein Vorfilter vorgelagert sein, welcher das Wasser filtert. Der Vorfilter kann eine Porengröße von 100 µm aufweisen. Als Filtermaterial für den Vorfilter kann ein Kunststoff verwendet werden, beispielsweise Polypropylen.

Die Vorrichtung weist eine Reinigungseinrichtung auf, welche dem Dampferzeuger vorgelagert ist und welche konfiguriert ist, in dem zugeführten Wasser gelöste Stoffe zumindest teilweise aus dem Wasser zu entfernen. Die Reinigungseinrichtung kann konfiguriert sein, die gelösten Stoffe mittels Umkehrosmose zu entfernen. Die Umkehrosmose ist ein Verfahren zur Aufkonzentrierung von in Flüssigkeiten gelösten Stoffen, bei der mit Druck der natürliche Osmose-Prozess umgekehrt wird. Das zugeführte Wasser, in dem die Konzentration eines Stoffes verringert werden soll, ist durch eine halbdurchlässige (semipermeable) Membran von einem Medium (beispielsweise ebenfalls Wasser) getrennt, in dem die Konzentration erhöht werden soll. Das Medium wird einem Druck ausgesetzt, der höher ist als der Druck, der durch das osmotische Verlangen zum Konzentrationsausgleich entsteht. Dadurch bewegen sich die Moleküle des Stoffes gegen ihre "natürliche" osmotische Ausbreitungsrichtung. Sie werden aus dem Wasser in das Medium gedrückt.

Es kann vorgesehen sein, dass die Reinigungseinrichtung der Enthärtungseinrichtung nachgelagert ist. Zwischen der Enthärtungseinrichtung und der Reinigungseinrichtung kann ein Feinfilter zum Filtern des Wassers angeordnet sein. Der Feinfilter kann eine Porengröße von 5 µm haben. Der Feinfilter kann aus einem Kunststoff bestehen, beispielsweise Polypropylen.

Es kann ein Wassertank zur Aufnahme des enthärteten und / oder gereinigten Wassers vorgesehen sein.

Mittels einer Kombination der Enthärtungseinrichtung und der Reinigungseinrichtung (ggf. unter Einbeziehung des Vorfilters und / oder des Feinfilters) kann eine Vollentsalzung des Wassers erzielt werden. Bei einer Vollentsalzung werden nicht nur die Härtebildner aus dem Wasser entfernt, sondern alle Ionen. Die Vollentsalzung kann mittels einer Kombination von Kationen- und Anionenaustauscher erreicht werden. Das vollentsalzte Wasser kann dem Dampferzeuger zugeführt werden, der hieraus Reinstdampf erzeugt.

Die Vorrichtung kann in einer Weiterbildung eine Trocknungseinrichtung aufweisen, welche dem Dampferzeuger nachgelagert ist und konfiguriert ist, den Wasserdampf zu trocken, bevor dieser in den Desorptionsbehälter eingeleitet wird. Feuchtigkeit wird aus dem Wasserdampf zumindest teilweise entfernt. Es kann vorgesehen sein, die entfernte Feuchtigkeit wieder dem Dampferzeuger zu zuführen. Die Trocknungseinrichtung kann in den Dampferzeuger integriert sein.

In einer weiteren Ausführungsform kann die Vorrichtung einen Pufferbehälter aufweisen, welcher die aus dem Kondensat abgeschiedenen halogenierten Kohlenwasserstoffe aufnimmt. Der Pufferbehälter kann mit dem Auffangbehälter und / oder dem weiteren Auffangbehälter verbunden sein. Der Pufferbehälter kann mit einer Füllstandskontrolle gebildet sein.

Die Vorrichtung kann des Weiteren eine Drucklufteinrichtung aufweisen, welche konfiguriert ist, die abgeschiedenen halogenierten Kohlenwasserstoffe aus dem Pufferbehälter in einen Transportbehälter zu befördern. Die Drucklufteinrichtung kann mit medizinisch reiner Luft betrieben werden.

Die Vorrichtung kann als geschlossenes System gebildet sein.

Die für die Vorrichtung offenbarten Merkmale können ebenso für ein Verfahren zum Betreiben einer derartigen Anlage relevant sein.

### Beschreibung beispielhafter Ausführungsformen

Im Folgenden werden beispielhafte Ausführungsformen unter Bezugnahme auf Figuren einer Zeichnung näher erläutert. Hierbei zeigen:
- Fig. 1: eine schematische Darstellung einer Vorrichtung und
- Fig. 2: eine schematische Darstellung einer weiteren Vorrichtung.

Im Folgenden werden für gleiche Komponenten gleiche Bezugszeichen verwendet.

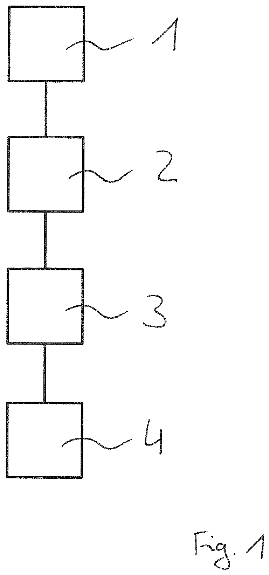

Fig. 1 zeigt eine schematische Darstellung einer Vorrichtung zur Rückgewinnung von Narkosegasen. Ein Sorbat, welches ein Zeolith mit adsorbiertem Narkosegas (z.B. Sevofluran) umfasst, wird in einen Desorptionsbehälter 2 gefüllt. Mittels eines Dampferzeugers 1 wird Wasserdampf erzeugt, der in den Desorptionsbehälter eingeleitet wird. Der Wasserdampf löst das Narkosegas von dem Zeolith und nimmt es auf. Der mit dem Narkosegas versetzte Wasserdampf wird in einen Kondensator 3 eingeleitet. In dem Kondensator 3 wird der Wasserdampf soweit gekühlt, sodass sich ein Kondensat bildet, welches in dem Auffangbehälter 4 gesammelt wird. In dem Kondensat setzt sich das Narkosegas am Boden des Auffangbehälters 4 ab, weil es schwerer als das Wasser ist. Das Wasser wird aus dem Auffangbehälter 4 abgeschöpft, sodass das kondensierte Narkosegas (Desorbat) gesammelt und erneut verwendet werden kann.

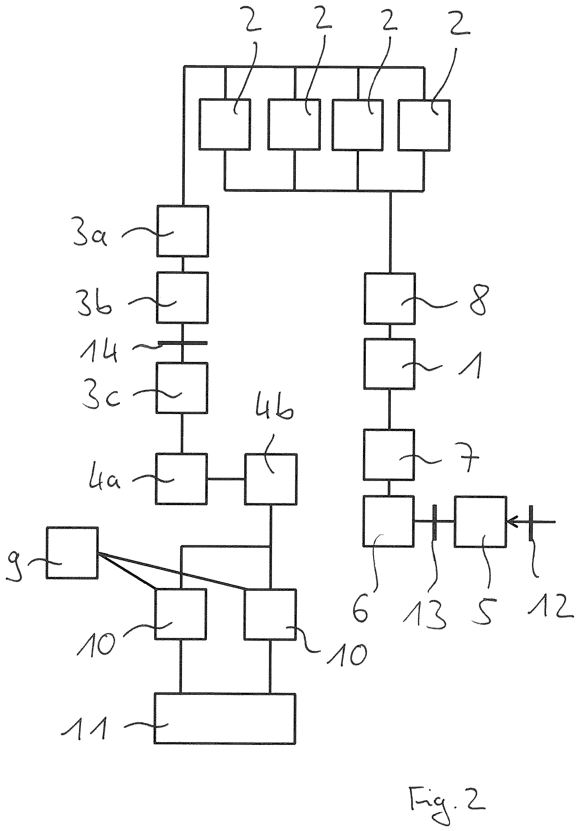

Fig. 2 zeigt eine weitere Ausführungsform der Vorrichtung. Die Vorrichtung weist folgende Komponenten auf: einen VE (Vollentsalzungs)-Bereich mit einer Enthärtungsanlage 5, einem Umkehrosmosemodul 6 und einem VE-Wasserbehälter 7, einen Reindampfbereich mit einem Reinstdampferzeuger 1 und einem Trockner 8, einem Kondensatbereich mit vier Desorptionsbehältern 2, zwei Edelstahlfilter (nicht dargestellt), einem Vorkühler 3a, einem Zwischenkühler 3b und einem Nachkühler 3c, einem Feinfilter 14, zwei Kondensatauffangbehältern 4 und zwei Pufferbehältern 10 sowie einen Druckluftbereich mit einer Druckluftanlage 9.

Der VE-Bereich umfasst die Enthärtungsanlage 5, das Umkehrosmosemodul 6 sowie den VE-Wasserbehälter 7. Die Enthärtungsanlage 5, welche die erste Stufe der VE-Wassererzeugung darstellt, ist an eine öffentliche Wasserversorgung (Stadtwasser) angeschlossen. Die zweite Stufe der Erzeugung von VE-Wasser stellt die Umkehrosmose dar. Zwischen dem Stadtwasseranschluss und der Enthärtungsanlage 5 sowie zwischen der Enthärtungsanlage 5 und Osmosemodul 6 sind Filter 12, 13 eingebaut. Der Vorfilter 12 hat eine Porengröße von 100 µm und als Filtermaterial wird Kunststoff verwendet, beispielsweise Polypropylen. Der Feinfilter 13 besitzt eine Porengröße von 5 µm und als Filtermaterial wird Polypropylen verwendet.

Der Dampferzeuger 1 weist 8 elektrische Heizelemente mit je 5 kW Leistung zur Erzeugung von Reinstdampf auf. Die Heizelemente werden abhängig vom Leistungsbedarf zur Regelung des Dampfdrucks einzeln zugeschaltet. Der Dampferzeuger 1 ist mit einer Füllstandskontrolle ausgestattet, welche einen oberen und einen unteren Schaltpunkt besitzt. Wenn der Füllstand den oberen oder den unteren Schaltpunkt erreicht, wird der Dampferzeuger 1 abgeschaltet. Um nur den trockenen Dampf zu den Desorptionsbehältern 2 zu zuführen, ist an den Dampferzeuger 1 ein Trockner 8 angeschlossen. Der Trockner 8 trennt die Feuchtigkeitsanteile vom trockenen Dampf und sorgt dafür, dass diese wieder der Wasserzuleitung am Dampferzeuger 1 zugeführt werden.

Die Vorrichtung ist mit vier identischen parallel angeordneten Desorptionsbehältern 2 ausgestattet, welche jeweils mit zwei Adsorbentienbehältern (nicht dargestellt) bestückt sind. Die Adsorbentienbehälter besitzen ein Volumen von ca. 20 1 und haben einen Innendurchmesser von 400 mm. Die Höhe der Desorptionbehälter 2 beträgt insgesamt 812 mm. Die Desorptionsbehälter 2 lassen sich manuell öffnen, um die Adsorbentienbehälter herauszunehmen. Die Adsorbentienbehälter lassen sich zur Befüllung öffnen. Dazu wird ein Tragegriff, der auf eine Gewindestange aufgeschraubt ist, von Hand abgeschraubt, sodass der Deckel abgenommen werden kann. Sowohl der Deckel als auch der Boden von jedem Adsorbentienbehälter sind mit einem Filtergewebe ausgestattet, welches eine Porengröße von 40 µm aufweist.

Die Edelstahlfilter mit einer Porengröße von 5 µm sind parallel angeordnet und können einzeln oder parallel betrieben werden. Dazu werden die entsprechenden Ventile am jeweiligen Filter geöffnet. Oberhalb und unterhalb vom Filter ist ein Ventil angeordnet. Dazu besitzt jeder Filter eine VE-Wasserzuleitung sowie eine Ableitung zum Abwasser. Zum Betrieb eines Filters werden beide Ventile unter- und oberhalb geöffnet. Des Weiteren können die Filter mit VE-Wasser aus dem VE-Wasserbehälter gespült werden. Zur Spülung eines Filters müssen die Ventile am Filter geschlossen werden. Dazu werden die jeweiligen Ventile an der VE-Wasserzuleitung sowie an der Ableitung geöffnet. Das VE-Wasser aus dem Spülprozess wird der Abwasserleitung zugeführt. Der verwendete Werkstoff der Filterkerze sowie für das Filtergehäuse ist Edelstahl.

Der Vorkühler 3a wird mit VE Wasser gespeist, um neu eingespeistes VE-Wasser für den Dampferzeuger 1 vorzuwärmen. Hinter dem Vorkühler ist ein Zwischenkühler 3b angeordnet, der mit Stadtwasser gespeist wird. Zwischen dem Zwischenkühler 3b und dem Nachkühler 3c ist ein zusätzlicher Filter 14 mit einer Porengröße von 5 µm angeordnet. Die Filterkerze des Filters 14 besteht aus Polypropylen und das Filtergehäuse ist aus Edelstahl (Werkstoffnummer 1.4404) gefertigt. Der Nachkühler 3c wird ebenfalls mit Stadtwasser als Kühlmedium betrieben.

Der Auffangbehälter 4a ist an den Nachkühler 3c angeschlossen und an der Unterseite mit einem weiteren Auffangbehälter 4b verbunden, welcher eine Füllstandskontrolle enthält. In dem Auffangbehälter 4a wird das Kondensat gesammelt. Dabei sinkt das Desorbat nach unten, weil es schwerer als Wasser ist. Auch im weiteren Auffangbehälter 4b wird das Desorbat getrennt vom leichteren Wasser gesammelt und bei Erreichen der entsprechenden Füllhöhe wahlweise von diesem in einen der Pufferbehälter 10 geleitet. Der weitere Auffangbehälter 4b ist mit dem Auffangbehälter 4a auch im oberen Bereich zusätzlich verbunden, um das Wasser wieder abzutrennen. Es besteht auch die Möglichkeit, das desorbierte Medium(z.B. für Probenentnahmen) über ein Ventil separat zu entnehmen.

Die Pufferbehälter 10 sind mit dem Auffangbehälter 4a und dem weiteren Auffangbehälter 4b verbunden. In den Pufferbehältern 10 können unterschiedliche Desorbate gelagert werden. Die Pufferbehälter 10 haben ein vorgesehenes Füllvolumen von 150 1. Beide Pufferbehälter 10 sind mit einer Füllstandskontrolle ausgestattet, welche auf der Füllhöhe entsprechend dem vorgesehenen Füllvolumen angebracht ist. Das Bruttovolumen der Behälter beträgt jeweils 220 1.

Im Schwarzraum befindet sich eine Druckluftversorgung, welche dazu dient, das Desorbat mittels Druckluft in einen Nachbarraum (Weißraum) über eine Rohrleitung in einen Transportbehälter 11 zu befördern. Hierzu wird medizinisch reine Luft verwendet, welche mit einem Adsorptionstrockner sowie einem Vor- und einem Nachfilter bereitgestellt wird. An beiden Pufferbehältern 10 sind Druckluftzuleitungen angeschlossen, um den nötigen Druck aufzubauen. Des Weiteren sind beide Behälter 10 mit der Transportleitung zum benachbarten Weißraum verbunden, um das Desorbat von einem der Pufferbehälter 10 in den Transportbehälter 11 zu füllen. Beide Behälter 10 sind an einen Sterilfilter angeschlossen. Dieser verhindert, dass Verunreinigungen aus der Umgebungsluft in das in den Pufferbehältern 10 befindliche Medium gelangen.

Die Vorrichtung hat folgende technische Daten:
- Produktionsleistung Enthärtung: max. 1000 Liter/h
- Produktionsleistung VE-Wasser: 40 Liter/h
- Produktionsleistung Reinstdampf: 60 kg/h bei 1,0 bar(Ü)
- elektrische Heizleistung: 40 kW
- Speisewasserverbrauch: max. 65 Liter/h

Es werden die folgenden Werkstoffe verwendet:
- produktberührte Teile: Edelstahl (Werkstoff-Nr. 1.4404 oder höherwertig)
- sonst Edelstahl (Werkstoff-Nr. 1.4301)
- Gestell aus Edelstahl - Rechteckprofile
- Außenverkleidung der Isolierung aus Edelstahl (Werkstoff-Nr. 1.4301)

Die in der Beschreibung, den Ansprüchen und den Figuren offenbarten Merkmale können sowohl einzeln als auch in beliebiger Kombination miteinander für die Verwirklichung der Erfindung relevant sein.

## Patentansprüche

1. Vorrichtung zur Rückgewinnung von halogenierten Kohlenwasserstoffen, mit:
- einem Desorptionsbehälter (2), der ein Sorbat aufnimmt, welches die halogenierten Kohlenwasserstoffe umfasst,
- einem Dampferzeuger (1), welcher konfiguriert ist, aus dem Dampferzeuger (1) zugeführten Wasser Wasserdampf zu erzeugen und den erzeugten Wasserdampf in den Desorptionsbehälter (2) einzuleiten, derart, dass die halogenierten Kohlenwasserstoffe von dem Sorbat desorbieren und von dem Wasserdampf aufgenommen werden,
- einer Kühleinrichtung (3), die konfiguriert ist, den mit halogenierten Kohlenwasserstoffen versetzten Wasserdampf zu kühlen, derart, dass ein Kondensat gebildet wird,
- einem Auffangbehälter (4), welcher das Kondensat aufnimmt, derart, dass die halogenierten Kohlenwasserstoffe nach unten sinken, und
- einem Pufferbehälter (10), welcher die aus dem Kondensat abgeschiedenen halogenierten Kohlenwasserstoffe aufnimmt,
**gekennzeichnet durch**
- eine Enthärtungseinrichtung (5), welche dem Dampferzeuger (1) vorgelagert ist und welche konfiguriert ist, den Härtegrad des zugeführten Wassers zu verringern,
- eine Reinigungseinrichtung (6), welche dem Dampferzeuger (1) vorgelagert ist und welche konfiguriert ist, in dem zugeführten Wasser gelöste Stoffe zumindest teilweise aus dem Wasser zu entfernen, und
- eine Drucklufteinrichtung (9), welche konfiguriert ist, die abgeschiedenen halogenierten Kohlenwasserstoffe aus dem Pufferbehälter (10) in einen Transportbehälter zu befördern, und
wobei die Kühleinrichtung (3) einen Vorkühler (3a) und einen Zwischenkühler (3b) aufweist, wobei der Vorkühler konfiguriert ist, neu eingespeistes Wasser für den Dampferzeuger (1) vorzuwärmen, und wobei der Zwischenkühler (3b) konfiguriert ist, den mit Kohlenwasserstoff versetzten Wasserdampf abzukühlen, so dass sich ein Kondensat bildet.

2. Vorrichtung nach Anspruch 1, des Weiteren eine Trocknungseinrichtung (8) aufweisend, welche dem Dampferzeuger (1) nachgelagert ist und konfiguriert ist, den Wasserdampf zu trocken, indem Feuchtigkeitsanteile von dem Wasserdampf getrennt werden, bevor dieser in den Desorptionsbehälter (2) eingeleitet wird.

3. Vorrichtung nach Anspruch 1 oder 2, aufweisend einen Nachkühler (3c), der in die Kühleinrichtung integriert ist, und nachfolgend dem Zwischenkühler angeordnet ist.

4. Vorrichtung nach einem der vorstehenden Ansprüche, aufweisend zusätzlich einen Filter (14), der zwischen dem Zwischenkühler (3b) und dem Nachkühler (3c) angeordnet ist.

5. Vorrichtung nach Anspruch 1 und umfassend die Merkmale der Ansprüche 2, 3 und 4, charakterisiert dadurch, dass:
a. die Reinigungseinrichtung (6) als Umkehrosmosemodul ausgebildet ist und
b. der Dampferzeuger (1) als Reinstdampferzeuger ausgebildet ist, sowie die Vorrichtung die folgenden Komponenten umfasst:
c. einen Vollentsalzungs-Wasserbehälter (7),
d. zwei Edelstahlfilter, die zwischen dem Desorptionsbehälter (2) und der Kühleinrichtung (3) angeordnet sind.

6. Vorrichtung nach einem der vorstehenden Ansprüche, aufweisend einen Vorfilter (12), der der Enthärtungseinrichtung (5) vorgelagert ist.

7. Vorrichtung nach einem der vorstehenden Ansprüche, aufweisend einen Feinfilter (13), der zwischen der Enthärtungseinrichtung (5) und der Reinigungseinrichtung (6) angeordnet ist.

## Claims

1. A device for recovering halogenated hydrocarbons, having:
- a desorption tank (2) holding a sorbate that contains the halogenated hydrocarbons,
- a steam generator (1) that is configured for generating steam from water supplied to the steam generator (1) and introducing the generated steam into the desorption tank (2) in such a way that the halogenated hydrocarbons are desorbed from the sorbate and absorbed by the steam,
- a cooling device (3) that is configured for cooling the steam combined with halogenated hydrocarbons in such a way that a condensate is formed,
- a collection tank (4) that holds the condensate in such a way that the halogenated hydrocarbons sink to the bottom, and
- a buffer tank (10) that holds the halogenated hydrocarbons that are separated from the condensate,
**characterized by**
- a water softening device (5) that is situated upstream from the steam generator (1) and that is configured for reducing the hardness of the supplied water,
- a cleaning device (6) that is situated upstream from the steam generator (1) and that is configured for at least partially removing dissolved substances from the supplied water, and
- a compressed air device (9) that is configured for conveying the separated halogenated hydrocarbons from the buffer tank (10) into a transport container,
wherein the cooling device (3) has a precooler (3a) and an intercooler (3b), the precooler being configured for preheating newly fed water for the steam generator (1), and the intercooler (3b) being configured for cooling the steam combined with hydrocarbons, thus forming a condensate.

2. The device according to Claim 1, also having a drying device (8) that is situated downstream from the steam generator (1) and configured for drying the steam by separating moisture components from the steam before it is introduced into the desorption tank (2).

3. The device according to Claim 1 or 2, having an aftercooler (3c) that is integrated into the cooling device and situated downstream from the intercooler.

4. The device according to one of the preceding claims, additionally having a filter (14) situated between the intercooler (3b) and the aftercooler (3c).

5. The device according to Claim 1 and comprising the features of Claims 2, 3, and 4, **characterized in that**:
a. the cleaning device (6) is designed as a reverse osmosis module and
b. the steam generator (1) is designed as a pure steam generator, and the device includes the following components:
c. a full desalination water tank (7),
d. two stainless steel filters situated between the desorption tank (2) and the cooling device (3).

6. The device according to one of the preceding claims, having a prefilter (12) situated upstream from the water softening device (5).

7. The device according to one of the preceding claims, having a fine filter (13) situated between the water softening device (5) and the cleaning device (6).

## Revendications

1. Dispositif de récupération d'hydrocarbures halogénés, comprenant :
- un récipient de désorption (2) qui reçoit un sorbat qui comprend les hydrocarbures halogénés,
- un générateur de vapeur (1) qui est conçu pour générer de la vapeur d'eau à partir de l'eau amenée au générateur de vapeur (1) et pour introduire la vapeur d'eau générée dans le récipient de désorption (2) de sorte que les hydrocarbures halogénés désorbent du sorbat et soient absorbés par la vapeur d'eau,
- un moyen de refroidissement (3) qui est conçu pour refroidir la vapeur d'eau, mélangée aux hydrocarbures halogénés, de manière à former un condensat,
- un récipient collecteur (4) qui reçoit le condensat de manière à faire descendre les hydrocarbures halogénés, et
- un récipient tampon (10) qui reçoit les hydrocarbures halogénés séparés du condensat,
**caractérisé par**
- un moyen d'adoucissement (5) qui est monté en amont du générateur de vapeur (1) et qui est conçu pour réduire le degré de dureté de l'eau amenée,
- un moyen de nettoyage (6) qui est monté en amont du générateur de vapeur (1) et qui est conçu pour éliminer au moins partiellement de l'eau les substances dissoutes dans l'eau amenée, et
- un moyen à air comprimé (9) qui est conçu pour transporter les hydrocarbures halogénés séparés du réservoir tampon (10) jusque dans un récipient de transport, et
le moyen de refroidissement (3) comportant un pré-refroidisseur (3a) et un refroidisseur intermédiaire (3b), le pré-refroidisseur étant conçu pour préchauffer l'eau nouvellement introduite pour le générateur de vapeur (1) et le refroidisseur intermédiaire (3b) étant conçu pour refroidir la vapeur d'eau mélangée aux hydrocarbures de manière à former un condensat.

2. Dispositif selon la revendication 1, comprenant en outre un moyen de séchage (8) qui est monté en aval du générateur de vapeur (1) et qui est conçu pour sécher la vapeur d'eau en séparant des fractions d'humidité de la vapeur d'eau avant que celle-ci ne soit introduite dans le récipient de désorption (2).

3. Dispositif selon la revendication 1 ou 2, comprenant un post-refroidisseur (3c) qui est intégré dans le moyen de refroidissement et qui est disposé en aval du refroidisseur intermédiaire.

4. Dispositif selon l'une des revendications précédentes, comprenant en outre un filtre (14) qui est disposé entre le refroidisseur intermédiaire (3b) et le post-refroidisseur (3c) .

5. Dispositif selon la revendication 1 et comprenant les particularités des revendications 2, 3 et 4, **caractérisé en ce que** :
a. le moyen de nettoyage (6) est réalisé comme un module à osmose inverse, et
b. le générateur de vapeur (1) est réalisé comme un générateur de vapeur pure, et
**en ce que** le dispositif comprend les composants suivants :
c. un récipient d'eau de déminéralisation totale (7),
d. deux filtres en acier inoxydable qui sont disposés entre le récipient de désorption (2) et le moyen de refroidissement (3).

6. Dispositif selon l'une des revendications précédentes, comprenant un pré-filtre (12) qui est monté en amont du moyen d'adoucissement (5).

7. Dispositif selon l'une des revendications précédentes, comprenant un filtre fin (13) qui est disposé entre le moyen d'adoucissement (5) et le moyen de nettoyage (6).
